Europäisches Patentamt
European Patent Office
Office européen des brevets

(19)

(11) Publication number: **0 021 692**
**A1**

(12) # EUROPEAN PATENT APPLICATION

(21) Application number: 80301921.5

(22) Date of filing: 09.06.80

(51) Int. Cl.[3]: C 07 C 79/46
C 07 C 121/38, C 07 D 213/30
C 07 C 149/18, C 07 C 147/02
C 07 D 307/44, A 01 N 33/22
A 01 N 43/40, A 01 N 39/02
A 01 N 41/10, A 01 N 43/08

(30) Priority: 11.06.79 US 47654
14.05.80 US 149618

(43) Date of publication of application:
07.01.81 Bulletin 81/1

(84) Designated Contracting States:
AT BE CH DE FR GB IT LI NL

(71) Applicant: Rohm and Haas Company
Independence Mall West
Philadelphia, Pennsylvania 19105(US)

(72) Inventor: Swithenbank, Colin
1710 West Rock Road
Perkasie, Pennsylvania(US)

(72) Inventor: Fujimoto, Ted
843 St. David's Avenue
Warminster, Pennsylvania, 18974(US)

(74) Representative: Wood, Peter Worsley Spencer et al,
Rohm and Haas Company Patent Department
Chesterfield House Barter Street
London, WC1A 2TP(GB)

(54) **Novel substituted nitrodiphenyl ethers, herbicidal compositions containing them and the use thereof for combating weeds.**

(57) Novel substituted nitrodiphenyl ethers, herbicidal compositions containing them and the use thereof for combating weeds.

Novel compounds are provided which exhibit activity as herbicides for combating weeds. The novel compounds have the formula:

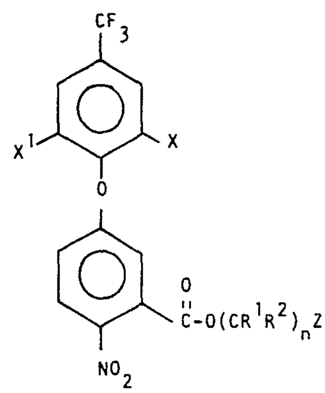

wherein X is hydrogen, halo, trihalomethyl, alkyl, cyano or nitro; $X^1$ is hydrogen, halo or trifluoromethyl; $R^1$ and $R^2$ are the same or different and are selected from hydrogen, $(C_1$-$C_4)$alkyl, phenyl and benzyl, the -$CR^1R^2$-groups being the same or different when n is $>1$; n is an integer of from 1 to 5; and Z is halo, nitro, cyano, trihalomethyl, methylthio, methylsulfinyl, methylsulfonyl, acetyl, phenyl or a heterocyclic group of from 4 to 6 nuclear atoms containing from 1 to 4 of the same or different nuclear heteroatoms selected from nitrogen, oxygen and sulfur.

EP 0 021 692 A1

Croydon Printing Company Ltd.

# Novel substituted nitrodiphenyl ethers, herbicidal compositions containing them and the use thereof for combating weeds

This invention concerns the provision of new nitrodiphenyl ethers, herbicidal compositions containing them and methods of combating weeds.

Diphenyl ethers are known to be effective weed control agents. See for example U.S. Patent Nos. 3,928,416; 3,454,392; 3,798,276; 3,873,303; 4,001,005 and 4,029,493 among the many patents issued. However, herbicidal effectiveness of a diphenyl ether cannot be predicted from its structure only. Often quite closely related compounds will have quite different weed control abilities. See, Advances in Agronomy, Vol. 24, pages 331, 332, 355, 356, 357 and 358, Herbicides, Chemical Degradation and Mode of Action, Kearney and Kaufman, Vol. 2, Dekker, Inc. pages 552-563 and 728-737 and Mode of Action of Herbicides, Ashton and Crafts and also U.S. Patent Nos. 3,454,392 and 3,776,961.

An ideal herbicide should give selective weed control, over the full growing season, with a single administration at low rates of application. It should be able to control all common weeds by killing them as the seed, the germinating seed, the seedling, and the growing plant. At the same time, the herbicide should be substantially nonphytotoxic to the crops to which it is applied and should decompose or otherwise be dissipated so as not to poison the soil permanently. The known diphenyl ether herbicides fall short of these ideals and thus the search has continued to discover new herbicides which are more selective or which complement the activities of known diphenyl ethers or other herbicides.

The new nitrodiphenyl ethers provided by the invention provide a useful choice in comparison with known nitrodiphenyl ether herbicides.

In accordance with the present invention, there is provided a new class of diphenyl ether herbicides of the following structural formula:

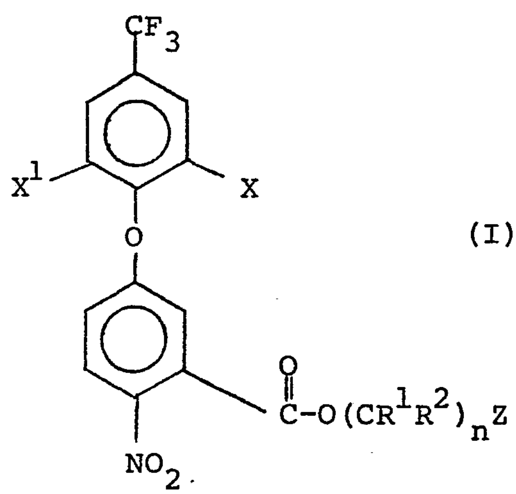

(I)

wherein X is hydrogen, halo, trihalomethyl (particularly trifluoromethyl), alkyl (for example, $(C_1-C_{10})$alkyl and particularly $(C_1-C_4)$alkyl such as methyl, ethyl, _n_-propyl, _n_-butyl and _tert_-butyl), nitro or cyano; $X^1$ is hydrogen, halo or trihalomethyl (particularly trifluoromethyl); $R^1$ and $R^2$ are the same or different and are selected from hydrogen, $(C_1-C_4)$alkyl, phenyl and benzyl, the $-CR^1R^2-$ groups being the same or different when n is >1; n is an integer of from 1 to 5; and Z is halo, trihalomethyl (particularly trifluoromethyl), nitro, cyano, methylthio, methylsulfinyl, methylsulfonyl, acetyl, phenyl or a heterocyclic group

having from 4 to 6 nuclear atoms containing from 1 to 3 or 4 of the same or different nuclear hetero atoms selected from nitrogen, oxygen and sulfur. Examples of heterocyclic groups are pyridyl, furyl, piperidyl, morpholinyl, thienyl thiazolyl, oxazolyl, oxadiazolyl, thiadiazolyl, tetrazolyl, pyridazinyl, pyrimidinyl, tetrahydrofuryl and tetrahydrothienyl. In preferred compounds X is halo (particularly chloro) and $X^1$ is hydrogen or halo (particularly chloro).

The term halo used throughout this specification means chloro, bromo, fluoro or iodo; preferred halo substituents are chloro or fluoro.

A sub-class of compounds are those of Formula I, wherein X (when alkyl) is $(C_1-C_4)$alkyl, Z is other than methylsulfinyl and when Z is a heterocyclic group such group contains 1 - 3 nuclear heteroatoms, eg groups such as pyridyl, furyl, piperidyl, morpholinyl or thienyl.

Examples of compounds embraced by Formula I include:

2-chloro-4-trifluoromethyl-3'-ω-chloro-n-propoxycarbonyl-4'-nitrodiphenyl ether;
2,6-dichloro-4-trifluoromethyl-3'-trifluoroethoxycarbonyl-4'-nitrodiphenyl ether;
2-fluoro-4-trifluoromethyl-3'-(α-methyl-α-nitro-n-butoxycarbonyl-4'-nitrodiphenyl ether;
4-trifluoromethyl-3'-(β-methyl-β-cyano-n-butoxycarbonyl-4'-nitrodiphenyl ether;
2-nitro-4-trifluoromethyl-3'-(β-phenethoxycarbonyl-4'-nitrodiphenyl ether;
2-cyano-4-trifluoromethyl-3'-(β-methylthioethoxycarbonyl)-4'-nitrodiphenyl ether;
2-methyl-4-trifluoromethyl-3'-(ε-methylsulphonyl-n-pentoxycarbonyl)-4'-nitrodiphenyl ether;
2,4-bis(trifluoromethyl)-3'-(ε-acetyl-n-butoxycarbonyl)-4'-nitrodiphenyl ether;

2-chloro-4-trifluoromethyl-3'-(furfuryloxycarbonyl)-4'-nitrodiphenyl ether;
4-trifluoromethyl-3'-(4-pyridyl)methoxycarbonyl-4'-nitrodiphenyl ether;
2,6-difluoro-4-trifluoromethyl-3'-{2-(3-pyrrollyl)ethoxycarbonyl}-4'-nitrodiphenyl ether;
2-chloro-4-trifluoromethyl-3'-{3-(2-pyrazinlyl)propoxycarbonyl}-4'-nitrodiphenyl ether;
2-chloro-4-trifluoromethyl-3'-{(2-thienyl)methoxycarbonyl}-4'-nitrodiphenyl ether;
2-chloro-4-trifluoromethyl-3'-{(3-thienyl)methoxycarbonyl}-4'-nitrodiphenyl ether;
2-chloro-4-trifluoromethyl-3'-{(2-thiazolyl)methoxycarbonyl}-4'-nitrodiphenyl ether;
2-chloro-4-trifluoromethyl-3'-{(4-thiazolyl)methoxycarbonyl}-4'-nitrodiphenyl ether;
2-chloro-4-trifluoromethyl-3'-{(5-thiazolyl)methoxycarbonyl}-4'-nitrodiphenyl ether;
2-chloro-4-trifluoromethyl-3'-{(2-oxazolyl)methoxycarbonyl}-4'-nitrodiphenyl ether;
2-chloro-4-trifluoromethyl-3'-{(4-oxazolyl)methoxycarbonyl}-4'-nitrodiphenyl ether;
2-chloro-4-trifluoromethyl-3'-{(5-oxazolyl)methoxycarbonyl}-4'-nitrodiphenyl ether;
2-chloro-4-trifluoromethyl-3'-{2-(1,3,4-oxadiazolyl)methoxycarbonyl}-4'-nitrodiphenyl ether;
2-chloro-4-trifluoromethyl-3'-{2-(1,3,4-thiadiazolyl)methoxycarbonyl}-4'-nitrodiphenyl ether;
2-chloro-4-trifluoromethyl-3'-{5-(1,2,3,4-tetrazolyl)methoxycarbonyl}-4'-nitrodiphenyl ether;
2-chloro-4-trifluoromethyl-3'-{(3-pyridazinyl)methoxycarbonyl}-4'-nitrodiphenyl ether;
2-chloro-4-trifluoromethyl-3'-{(4-pyridazinyl)methoxycarbonyl}-4'-nitrodiphenyl ether;
2-chloro-4-trifluoromethyl-3'-{(2-pyrimidinyl)methoxycarbonyl}-4'-nitrodiphenyl ether;

2-chloro-4-trifluoromethyl-3'-{(4-pyrimidinyl)methoxycarbonyl}-4'-nitrodiphenyl ether;
2-chloro-4-trifluoromethyl-3'-{(5-pyrimidinyl)methoxycarbonyl}-4'-nitrodiphenyl ether;
2-chloro-4-trifluoromethyl-3'-{(2-pyrazinyl)methoxycarbonyl}-4'-nitrodiphenyl ether;
2-chloro-4-trifluoromethyl-3'-{(2-tetrahydrofurfuryl)methoxycarbonyl}-4'-nitrodiphenyl ether;
2-chloro-4-trifluoromethyl-3'-{(3-tetrahydrofurfuryl)methoxycarbonyl}-4'-nitrodiphenyl ether;
2-chloro-4-trifluoromethyl-3'-{(2-tetrahydrothienyl)methoxycarbonyl}-4'-nitrodiphenyl ether;
2-chloro-4-trifluoromethyl-3'-{(3-tetrahydrothienyl)methoxycarbonyl}-4'-nitrodiphenyl ether;
2-chloro-4-trifluoromethyl-3'-{(2-acetyl)ethoxycarbonyl}-4'-nitrodiphenyl ether; and
2-chloro-4-trifluoromethyl-3'-{3-(2-oxo)tetrahydrofurfuryloxycarbonyl}-4'-nitrodiphenyl ether;
2-trichloromethyl-4-trifluoromethyl-3'-(2-chloroethoxycarbonyl)-4'-nitrodiphenyl ether;
2-tribromomethyl-4-trifluoromethyl-3'-(2-chloroethoxycarbonyl)-4'-nitrodiphenyl ether;
2-n-butyl-4-trifluoromethyl-3'-(2-chloroethoxycarbonyl)-4'-nitrodiphenyl ether;
2-n-decyl-4-trifluoromethyl-3'-(2-chloroethoxycarbonyl)-4'-nitrodiphenyl ether;
6-trifluoromethyl-4-trifluoromethyl-3'-(2-chloroethoxycarbonyl)-4'-nitrodiphenyl ether;
6-trichloromethyl-4-trifluoromethyl-3'-(2-chloroethoxycarbonyl)-4'-nitrodiphenyl ether;
6-tribromomethyl-4-trifluoromethyl-3'-(2-chloroethoxycarbonyl)-4'-nitrodiphenyl ether;
2-chloro-4-trifluoromethyl-3'-(2-cyanohexyloxycarbonyl)-4'-nitrodiphenyl ether;
2-chloro-4-trifluoromethyl-3'-(2-nitro-2-phenylethoxycarbonyl)-4'-nitrodiphenyl ether;

2-chloro-4-trifluoromethyl-3'-(1-benzyl-2-chloroethoxycarbonyl)-4'-nitrodiphenyl ether.

The diphenyl ethers of this invention may be prepared by any suitable method described in the literature for the preparation of compounds having an analogous structure or by other methods devised for the purpose. A first method involves reacting an appropriate acid halide with an appropriate hydroxy-substituted compound. A second method involves reacting an appropriate alkali metal or alkaline earth metal carboxylate with an appropriate halo-substituted compound.

In the first method a 4-trifluoromethylphenyl-3-halocarbonyl-4-nitrophenyl ether is reacted with an hydroxy compound in the presence of an inert solvent such as toluene. This reaction may be conducted at a temperature in the range of from about 20 to about 150°C. Reaction times may vary from about 15 minutes to about 16 hours. The reaction is usually conducted at a temperature between 50 to 100°C for a period of time of about 1/2 to about 2 hours. The following reaction scheme applies to this method:

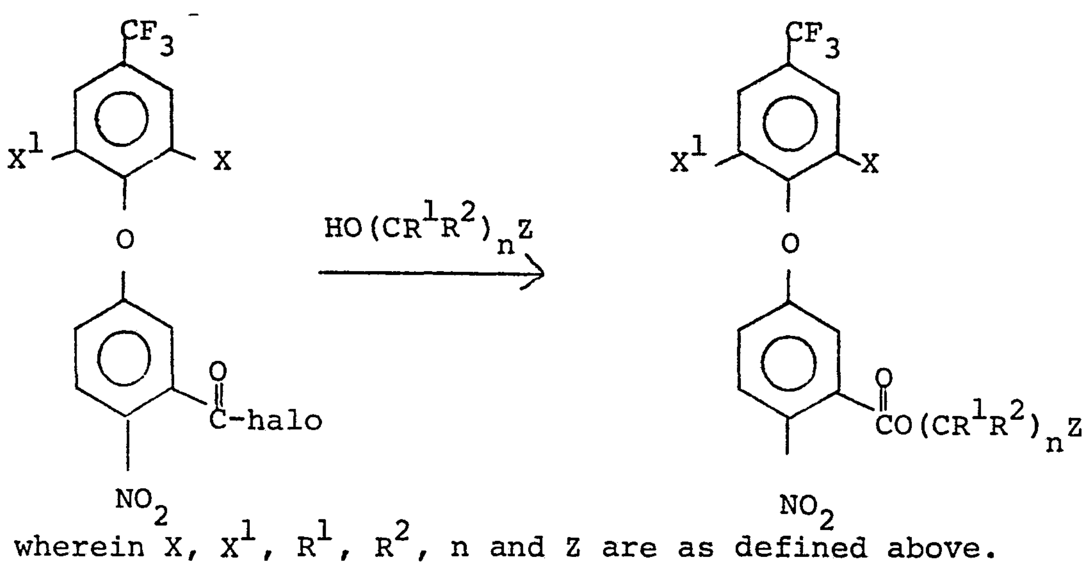


wherein X, $X^1$, $R^1$, $R^2$, n and Z are as defined above.

In the second method a 4-trifluoromethylphenyl-3-alkali

metal (or alkaline earth metal) oxycarbonyl-4-nitrophenyl ether is reacted with an appropriate halo-substituted compound in the presence of an inert solvent (eg dimethyl formamide, dimethyl sulfoxide or methyl ethyl ketone) at a temperature in the range of from about 20 to about 150°C. The reaction period may be in the range of from about 1/4 to about 16 hours. The following reaction scheme illustrates this method:

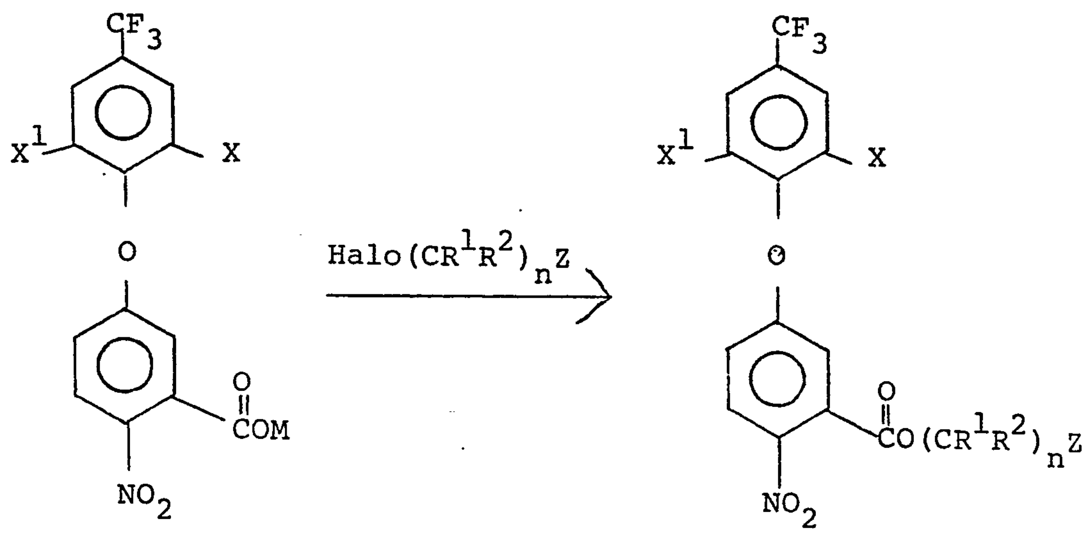


wherein X, $X^1$, $R^1$, $R^2$, n and Z are as defined above and M is a cation derived from an alkali metal or alkaline earth metal.

The diphenyl ethers of Formula I are useful as preemergence and postemergence herbicides. Preemergence herbicides are ordinarily used to treat the soil by application either before seeding, during seeding, after seeding and before the crop emerges. Postemergence herbicides are those which are applied after the plants have emerged and during their growth period. The compounds of Formula I are especially active as postemergence herbicides. Thus the invention provides a method of combating weeds, usually in an agronomic crop area, which comprises applying to the growing weeds, eg weed seedlings, or to a growth medium prior to emergence of the weeds, one or more of the diphenyl ethers in an amount sufficient to combat the growth of the weeds.

Among the crops on which the diphenyl ethers of Formula I can be advantageously employed are cotton, soybeans, peanuts, beans, peas, carrots, maize, wheat and other cereal crops.

The diphenyl ethers of Formula I are useful for controlling weeds in rice crops. When used in transplanted rice crops, the ethers can be applied either preemergence or postemergence to the weeds -- that is, they can be applied to the transplanted rice plants and their growth medium either before the weed plants have emerged or while they are in their early stages of growth. The ethers can be applied to the growth medium either before or after the rice has been transplanted to that medium.

The diphenyl ethers can be applied in any amount which will give the required control of weeds. A standard rate of application of the herbicides of the invention is in the range from 0.1 to 13.5 kg of diphenyl ether per hectare. A preferred range is from 0.1 to 2.24 kg/ha.

Under some conditions, the diphenyl ethers may be advantageously incorporated into the soil or other growth medium prior to planting a crop. This incorporation can be by any convenient means, including simple mixing with the soil, applying the diphenyl ether to the surface of the soil and then discing or dragging into the soil to the desired depth, or by employing a liquid carrier.

The diphenyl ethers of the invention can be applied to the growth medium or to plants to be treated either neat or as a component in a herbicidal composition or formulation which also comprises an agronomically acceptable carrier. "Agronomically acceptable carrier" is any carrier which can be used to dissolve, disperse or diffuse a herbicidal compound in the composition without impairing the effectiveness of

the herbicidal compound and which by itself has no permanent detrimental effect on the soil, equipment, crops, or agronomic environment. Mixtures of the diphenyl ethers of the invention may also be used in any of these herbicidal formulations.

Herbicidal formulations may be in the form of (a) a dust or granules or (b) a formulation which contains a surfactant and which is a wettable powder, concentrated liquid suspension, solution, water-dispersible paste, emulsifiable concentrate or flowable emulsion concentrate. In general the diphenyl ethers may be formulated and used as described in Australian Published Patent Application No. 506,509 and they may be used in conjunction with other pesticides as described in said Australian Application.

The following examples are given by way of illustration. Percentages are on a weight basis unless otherwise noted.

Example 1

2-Chloro-4-trifluoromethyl-3'-(2-chloroethoxycarbonyl)-4'-nitrodiphenyl ether

To a 1 necked flask equipped with a stirrer was added 2-chloro-4-trifluoromethyl-3'-chlorocarbonyl-4'-nitrophenyl ether (19.0 g; 0.05 moles), 2-chloroethanol (40 g; 0.5 moles) and toluene (100 ml). The solution was heated at reflux overnight. A sample taken for gas liquid chromotography analysis indicated complete reaction. The solvent and excess 2-chloroethanol were removed on a rotatory evaporator to afford a gum which was dissolved in hexane and washed with dilute potassium carbonate solution and then with water. The hexane solution was dried over anhydrous magnesium sulfate and then filtered through activated silica gel. The solvent was removed to afford 22 g of 2-chloro-4-trifluoromethyl-3'-(2-chloroethoxycarbonyl)-4'-nitrodiphenyl ether, melting point 58-61$^{o}$C. Elemental Analysis

for $C_{16}H_{10}Cl_2F_3N_1O_5$: Calculated: C, 45.30; H, 2.38; N, 3.30; Cl, 16.72; F, 13.44; Found: C, 45.18; H, 2.42; N, 3.19; Cl, 16.94; F, 12.76. $^1$H nmr 3' side chain Δ($CDCl_3$) 3.74(t,2H), 4.5(t,2H).

Example 2

2-Chloro-4-trifluoromethyl-3'-(2-nitroethoxycarbonyl)-4'-nitrodiphenyl ether

A solution of 2-chloro-4-trifluoromethyl-3'-chlorocarbonyl-4'-nitrodiphenyl ether (19.0 g; 0.05 moles) and 2-nitroethanol (9.0 g; 0.1 mole) in toluene (100 ml) was refluxed overnight and the solvents removed under reduced pressure to afford 18 g of 2-chloro-4-trifluoromethyl-3'-(2-nitroethoxycarbonyl)-4'-nitrodiphenyl ether. Elemental Analysis for $C_{16}H_{10}ClF_3N_2O_7$; Calculated: C, 44.20; H, 2.32; N, 6.44; Cl, 8.15 and F, 13.11; Found: C, 44.56; H, 2.28; N, 6.50: Cl, 8.46 and F, 12.60. $^1$H nmr 3' side chain Δ($CDCl_3$) 3.74(t,2H), 4.78(s,4H).

Example 3

2-Chloro-4-trifluoromethyl-3'-(2-pyridylmethoxycarbonyl)-4'-nitrodiphenyl ether

A solution of 2-chloro-4-trifluoromethyl-3'-chlorocarbonyl-4'-nitrodiphenyl ether (19.0 g; 0.05 moles) and 2-pyridylcarbinol (10.9 g; 0.1 moles) in toluene (100 ml) was refluxed overnight. The procedure of Example 1 was followed in working up the reaction mixture to obtain 22 g of 2-chloro-4-trifluoromethyl-3'-(2-pyridylmethoxycarbonyl)-4'-nitrodiphenyl ether. Elemental Analysis for $C_{20}H_{12}ClF_3N_2O_5$; Calculated: C, 52.93; H, 2.89; N, 6.17; Cl, 7.81; F, 12.56; Found: C, 52.57; H, 2.82; N, 6.16; Cl, 8.43; F, 11.97. $^1$H nmr 3' side chain Δ($CDCl_3$) 3.74(t,2H), 4.46(s,2H).

Example 4

2-Chloro-4-trifluoromethyl-3'-(cyanomethoxycarbonyl)-4'-nitrodiphenyl ether

2-Chloro-4-trifluoromethyl-3-carboxy-4'-nitrodiphenyl ether (18.05 g; 0.05 moles) and potassium carbonate (anhydrous; 6.9 g) in dimethylsulfoxide (100 ml) was heated to 100°C with stirring. The reaction mixture was cooled to 40°C and chloroacetonitrile (4.0 g) was added and the reaction mixture heated again to 100°C with stirring. Stirring was continued for 2 hours and then the reaction mixture was cooled, diluted with water and extracted with diethyl ether. The extract was diluted with hexane and washed with aqueous 1% sodium hydroxide solution. The extract was dried, filtered through activated silica gel and the solvents removed under reduced pressure to afford 13.0 g of 2-chloro-4-trifluoromethyl-3'-cyanomethoxycarbonyl-4'-nitrodiphenyl ether as a pale yellow oil. Elemental Analysis for $C_{16}H_8N_2ClF_3O_5$: Calculated: C, 47.96; H, 2.01; N, 6.99; Cl, 8.85; F, 14.22; Found: C, 47.52; H, 1.82; N, 6.73; Cl, 8.75; F, 14.60. $^1H$ nmr 3' side chain $\Delta(CDCl_2)$ 3.74(t,2H), 5.0(s,2H).

Example 5

2-Chloro-4-trifluoromethyl-3'-(methylthiomethoxycarbonyl)-4'-nitrodiphenyl ether

To a methylethyl ketone solution of 2-chloro-4-trifluoromethyl-3'-carboxy-4'-nitrodiphenyl ether (72.3 g; 0.20 mole) at 25°C was added potassium carbonate (27.6 g; 0.20 mole). The mixture was heated to 60°C for 10 minutes. Chloromethylmethylsulfide (19.3 g; 0.20 mole) was then added and the emulsion stirred 3 hours at 65-75°C. The emulsion was diluted with ether, washed with water, and aqueous potassium carbonate solution, dried with molecular sieves and filtered through activated silica gel. Removal of the solvent afforded 2-chloro-4-trifluoromethyl-3'-(methylthiomethoxycarbonyl)-4'-nitrodiphenyl ether as an oil (48.5 g, 58.5%), 90% pure by nmr. $^1H$ n.m.r. of 3' side-chain $\Delta(CDCl_3)$ 2.25 (s, 3H), 5.35 (s, 2H). IR ($cm^{-1}$) 1725 (carbonyl), 700 (C-S). Elemental Analysis calculated for $C_{16}H_{11}ClF_3N_1O_5S_1$: Found:

C, 45.71; H, 2.84; N, 3.04; Calculated: C, 45.55; H, 2.63; N, 3.32.

Example 6

2-Chloro-4-trifluoromethyl-3'-(methylsulfinylmethoxycarbonyl)-4'-nitrodiphneyl ether

To a chloroform solution of 2-chloro-4-trifluoromethyl-3'-(methylthiomethoxycarbonyl)-4'-nitrodiphenyl ether (10.5 g; 0.02 mole) at 25°C was added a chloroform solution of m-chloroperoxybenzoic acid (4.06 g; 0.02 mole). The solution was stirred overnight at 25°C and then for one hour under reflux. After cooling, the solution was washed with water, aqueous potassium carbonate and sodium sulfite, and dried by molecular sieves. After filtering through activated silica gel and stripping, an oily solid was obtained which was recrystallized from chloroform-hexane to afford 2-chloro-4-trifluoromethyl-3'-(methylsulfinylmethoxycarbonyl)-4'-nitrodiphenyl ether white crystals (1.7 g, 19.3%) m.p. 130-132°C, 90% pure by nmr. Elemental Analysis calculated for $C_{16}H_{11}Cl_1F_3N_1O_6S_1$: Found: C, 42.52; H, 2.58; N, 3.00; Calculated: C, 43.89; H, 2.53; N, 3.20.

Example 7

2-Chloro-4-trifluoromethyl-3'-(methylsulfonylmethoxycarbonyl)-4'-nitrodiphenyl ether

To a chloroform solution of 2-chloro-4-trifluoromethyl-3'-(methylthiomethoxycarbonyl)-4'-nitrodiphenyl ether (9.6 g; 0.023 mole) at 25°C was added a chloroform solution of m-chloroperoxybenzoic acid (18.5 g; 0.091 mole) and the solution was stirred overnight at 25°C, then for 1 hour at 40°C. The solution was then washed with water, aqueous potassium carbonate and sodium sulfite solutions. After drying with molecular sieves, the solution was filtered through activated silica gel and stripped to afford 2-chloro-4-trifluoromethyl-3'-(methylsulfonylmethoxycarbonyl)-4'-nitrodiphenyl ether as an oil (1.8 g, 17.3%), 74% by

nmr. $^{1}H$ n.m.r. of 3' side-chain $\Delta(CDCl_3)$ 2.95 (s, 3H), 5.28 (s, 2H). IR ($cm^{-1}$) 1759 (carbonyl), 1125 and 1320 ($SO_2$). Elemental Analysis calculated for $C_{16}H_{11}Cl_1F_3N_1O_7S_1$ Found: C, 42.88; H, 2.62; N, 2.38; Calculated: C, 42.34; H, 2.44; N, 3.09.

Example 8

2-Chloro-4-trifluoromethyl-3-{α-(acetyl)ethoxycarbonyl}-4'-nitrodiphenyl ether

To a methylethyl ketone solution of 2-chloro-4-trifluoromethyl-3'-carboxy-4'-nitrodiphenyl ether (14.5 g; 0.04 mole) at 25°C was added potassium carbonate (11.04 g; 0.08 mole). The mixture was stirred for 20 minutes. To this mixture there was then added a methylethyl ketone solution of 3-bromo-2-butanone (6.04 g; 0.04 mole) and stirring was carried out for 3 hours at 25°C. The mixture was diluted with ether, washed with water, then with an aqueous solution of sulfuric acid and finally with an aqueous solution of potassium carbonate. After drying with molecular sieves, the solution was filtered through activated silica gel and on removal of the solvent afforded 2-chloro-4-trifluoromethyl-3-{α(acetyl)ethoxycarbonyl}-4'-nitrodiphenyl ether as an oil (14.1 g, 81.6%) 90% pure by nmr. $^{1}H$ n.m.r. of 3' side chain $\Delta(CDCl_3)$ 1.58 (d, 3H), 2.28 (d, 3H), 5.38 (quart., 1H). IR ($cm^{-1}$) 1740 (carbonyl). Elemental Analysis calculated for $C_{18}H_{13}Cl_1F_3N_1O_6$: Found: C, 48.55; N, 3.23; Cl, 9.00; F, 12.52; H, 2.96; Calcd: C, 50.07; N, 3.24; Cl, 8.21; F, 13.20; H, 3.03.

Example 9

2-Chloro-4-trifluoromethyl-3'-(furfuryloxycarbonyl)-4'-nitrodiphenyl ether

To a refluxing toluene solution of 2-chloro-4-trifluoromethyl-3'-carboxy-4'-nitrodiphenyl ether (14.5 g; 0.04 mole) there was added a toluene solution of furfuryl alcohol (4.5 g; 0.045 mole). The solution was heated at

reflux for 10 hours. The mixture was diluted with hexane and washed with water and successively with aqueous solutions of sodium carbonate and brine. The organic phase was then treated with activated carbon, dried with magnesium sulfate filtered first through activated silica gel and then through basic alumina. Removal of the solvent afforded 2-chloro-4-trifluoromethyl-3'-(furfuryloxycarbonyl) 4'-nitrodiphenyl ether as an oil (4.7 g; 26.6%), 90% pure by nmr. $^1H$ n.m.r. of 3' side-chain $\Delta(CDCl_3)$ 5.38 (s, 1H), 6.3-6.58 (m, 6H). IR ($cm^{-1}$) 1740 (carbonyl). Elemental Analysis calculated for $C_{19}H_{11}Cl_1F_3N_1O_6$: Found: C, 53.13; H, 2.69; N, 2.98; Cl, 7.60; F, 10.85; Calculated: C, 51.65; H, 2.51; N, 3.17; Cl, 8.03; F, 12.90.

In a manner similar to that described in Example 1 or 4 all of the diphenyl ethers of this invention may be obtained. The diphenyl ethers identified in Table I below are, by following substantially the procedure of Example 1 or 4, also prepared in accordance with the reaction scheme preceding Table I.

Table I

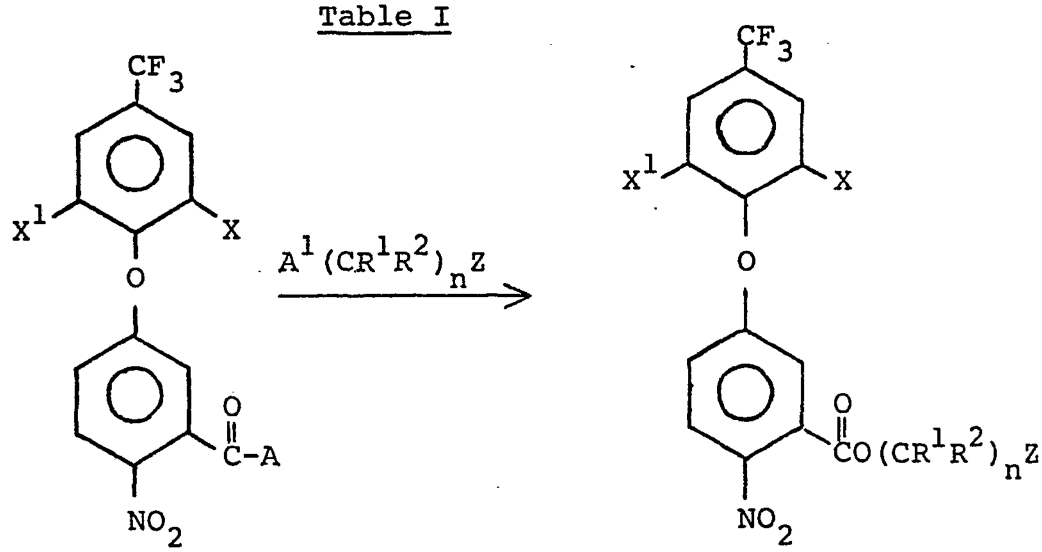

Table I (continued)

| Ex. No. | X | $X^1$ | A | $A^1$ | $R^1$ | $R^2$ | n | Z |
|---|---|---|---|---|---|---|---|---|
| 10 | Cl | Cl | Cl | OH | H | $CH_3$ | 2 | F |
| 11 | Cl | H | Cl | OH | H | H | 2 | Cl |
| 12 | $CF_3$ | H | ONa | Cl | H | H | 3 | 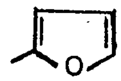 |
| 13 | $CF_3$ | H | ONa | Br | H | H | 4 | 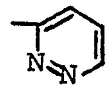 |
| 14 | CN | H | Cl | OH | $CH_3$ | $CH_3$ | 2 | 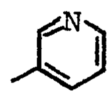 |
| 15 | F | Br | ONa | Br | H | H | 5 | $NO_2$ |
| 16 | $CH_3$ | H | Cl | OH | $CH_3$ | $CH_3$ | 3 | 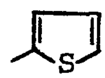 |
| 17 | Cl | H | Cl | OH | H | H | 3 | $CF_3$ |

Herbicidal Activity

Many of the diphenyl ethers of the foregoing Examples were evaluated on the following representative species:

| | | | Approx. No. Seeds |
|---|---|---|---|
| Monocots | Barnyardgrass | (*Echinochloa crusgalli*) | 25 |
| | Downybrome | (*Bromus tectorum*) | 20 |
| | Foxtail | (*Setaria* spp) | 25 |
| | Johnsongrass | (*Sorghum halepense*) | 25 |
| | Wild Oat | (*Avena fatua*) | 20 |
| | Nutsedge | (*Cyperus esculentus*) | 5 |
| Dicots | Cocklebur | (*Xanthium pensylvanicum*) | 3 |
| | Marigold | (*Tagetes* spp) | 15 |
| | Morningglory | (*Ipomoea* spp) | 10 |
| | Tomato | (*Lycopersicon esculentum*) | 15 |
| | Velvetleaf | (*Abutilon theophrasti*) | 15 |

Test Procedure

Seeds of the above species were planted in soil in trays (approx. 18 cm x 27 cm x 8 cm). For preemergence tests,

the trays were sprayed with the test compound immediately after planting. For postemergence tests, the seeds were allowed to germinate and after growing in the greenhouse for two weeks, the growing plants were treated with the test compound. The compound to be evaluated was dissolved in acetone, water or a mixture thereof and sprayed over the trays using a carrier volume equivalent to 468 l/ha (50 US gallons per acre) at the rate of application (in kg/ha) specifed in Table II below. About two weeks after application of the test compound, the state of growth of the plants was observed and the phytotoxic effect of each compound determined as follows: each species was evaluated on a scale of 0-100 in which 0 = no activity and 100 = total kill and the results for the monocots and dicots were separately averaged. Table II shows the results obtained for the compounds of the invention at rates of 0.56 kg/ha (0.5 lb/A) and 2.24 kg/ha (2.0 lb/A). In Table II "AM" means average for monocots and "AD" means average for dicots.

Table II

| Compound of Example No. | 0.56 kg/ha | | | | 2.24 kg/ha | | | |
|---|---|---|---|---|---|---|---|---|
| | Pre | | Post | | Pre | | Post- | |
| | AM | AD | AM | AD | AM | AD | AM | AD |
| 1 | - | - | - | - | 76 | 97 | 43 | 66 |
| 2 | - | - | - | - | 81 | 92 | 30 | 97 |
| 3 | - | - | - | - | 52 | 64 | 23 | 70 |
| 4 | 72 | 72 | 18 | 80 | 94 | 99 | 42 | 99 |
| 5 | 45 | 96 | 32 | 80 | 77 | 93 | 53 | 100 |
| 6 | 52 | 84 | 18 | 100 | 77 | 99 | 47 | 100 |
| 7 | 43 | 80 | 20 | 80 | 33 | 72 | 30 | 78 |
| 8 | 38 | 56 | 5 | 52 | 53 | 98 | 20 | 86 |
| 9 | 55 | 99 | 30 | 76 | 87 | 98 | 47 | 86 |

Claims:

1. A compound of the formula

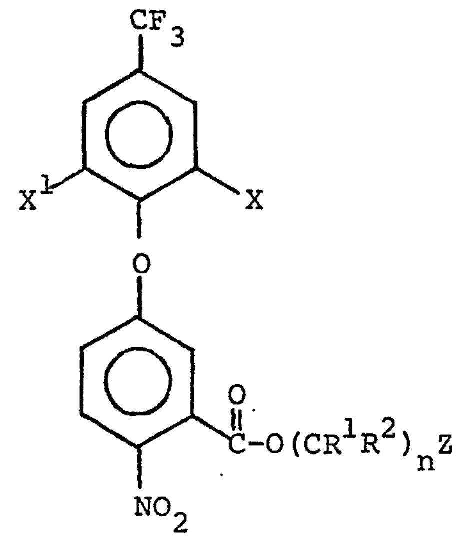

wherein X is hydrogen, halo, trihalomethyl, alkyl, cyano or nitro;

$X^1$ is hydrogen, halo or trifluoromethyl;

$R^1$ and $R^2$ are the same or different and are selected from hydrogen, $(C_1-C_4)$alkyl, phenyl and benzyl, the $-CR^1R^2-$ groups being the same or different when n is >1;

n is an integer of from 1 to 5; and

Z is halo, nitro, cyano, trihalomethyl, methylthio, methylsulfinyl, methylsulfonyl, acetyl, phenyl or a heterocyclic group of from 4 to 6 nuclear atoms containing from 1 to 4 of the same or different nuclear heteroatoms selected from nitrogen, oxygen and sulfur.

2. A compound as claimed in Claim 1, wherein X (when alkyl) is $(C_1-C_4)$alkyl.

3. A compound as claimed in Claim 1, wherein X (when alkyl) is $C_1-C_4$ alkyl, Z is other than methylsulfinyl and when Z is a heterocyclic group such group contains 1 to 3 nuclear heteroatoms.

4. A compound as claimed in Claim 3, wherein Z (when a heterocyclic group) is pyridyl, furyl, piperidyl, morpholinyl or thienyl.

5. A compound as claimed in Claim 1, wherein X is hydrogen, chloro, trifluoromethyl, methyl or cyano; $X^1$ is hydrogen, chloro or trifluoromethyl; $R^1$ and $R^2$ are the same or different and are selected from hydrogen or methyl; n is an integer of from 1 to 5 and Z is halo, nitro, cyano, trifluoromethyl or a heterocyclic group selected from pyridyl, furyl, piperidyl, morpholinyl, thienyl, thiazolyl, oxazolyl, oxadiazolyl, thiadiazolyl, tetrazolyl, pyridazinyl, pyrimidinyl, tetrahydrofuryl or tetrahydrothienyl.

6. A compound as claimed in Claim 1 which is 2-chloro-4-trifluoromethyl-3'-(2-chloroethoxycarbonyl)-4'-nitrodiphenyl ether, 2-chloro-4-trifluoromethyl-3'-(2-nitroethoxycarbonyl)-4'-nitrodiphenyl ether, 2-chloro-4-trifluoromethyl-3'-(2-pyridylmethoxycarbonyl)-4'-nitrodiphenyl ether, 2-chloro-4-trifluoromethyl-3'-(cyano-methoxycarbonyl)-4'-nitrodiphenyl ether or 2-chloro-4-trifluoromethyl-3'-(furfuryloxycarbonyl)-4'-nitrodiphenyl ether.

7. A compound as claimed in Claim 1 which is 2-chloro-4-trifluoromethyl-3'-(methylthiomethoxycarbonyl)-4'-nitrodiphenyl ether, 2- chloro-4-trifluoromethyl-3'-(methylsulfinylmethoxycarbonyl)-4'-nitrodiphenyl ether, 2-chloro-4-trifluoromethyl-3'-(methylsulfonylmethoxy-carbonyl)-4'-nitrodiphenyl ether, 2-chloro-4-trifluoro-methyl-3-{α-(acetyl)ethoxycarbonyl}-4'-nitrodiphenyl ether.

8. A method of combating weeds which comprises applying to growing weeds or to a growth medium prior to emergence of weeds therefrom a compound as claimed in any

one of Claims 1 to 7 in an amount sufficient to combat the growth of the weeds.

9. A herbicidal composition containing, as active ingredient, at least one compound as claimed in any one of Claims 1 to 7 and an agronomically acceptable carrier or diluent for the active ingredient, the composition also optionally containing a surfactant.

10. A composition as claimed in Claim 9 in the form of (a) a dust or granules or (b) a formulation which contains a surfactant and which is a wettable powder, concentrated liquid suspension, solution, water-dispersible paste, emulsifiable concentrate or flowable emulsion concentrate.

0021692

European Patent Office

# EUROPEAN SEARCH REPORT

Application number

EP 80 30 1921

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| A | US - A - 4 031 131 (W.O. JOHNSON)<br>* Column 1, lines 1-39; columns 5,6; example 3 *<br>-- | 1 | C 07 C 79/46<br>121/38<br>C 07 D 213/30<br>C 07 C 149/18<br>147/02<br>C 07 D 307/44<br>A 01 N 33/22<br>43/40<br>39/02<br>41/10<br>43/08 |
| A | US - A - 4 093 446 (H.O. BAYER)<br>* Column 1, line 48 - column 2, line 27; columns 13,14; example 49 *<br>-- | 1,8 | TECHNICAL FIELDS SEARCHED (Int.Cl. 3) |
| A | DE - A - 2 753 900 (ROHM AND HAAS)<br>* Pages 1,2; claim 1; page 3; claims 10,11 *<br>---- | 1,8 | C 07 C 79/46 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| | The present search report has been drawn up for all claims | | &: member of the same patent family, corresponding document |

| Place of search | Date of completion of the search | Examiner |
|---|---|---|
| The Hague | 18-09-1980 | KINZINGER |

EPO Form 1503.1 06.78